# EUROPEAN PATENT APPLICATION

(11) **EP 4 696 242 A1**
(43) Date of publication of application: **18.02.2026**
(21) Application number: 25196183.5
(22) Date of filing: 15.08.2025
(51) Int. Cl.: A61B 6/51

(54) **MULTI BEAM X-RAY IMAGING**

(30) Priority: 16.08.2024 US 202418807183
(71) Applicant: DENTSPLY SIRONA Inc., York, PA 17401 (US)
(72) Inventor: ELVERS, Michael, 64625 Bensheim (DE); MAUR, Susanne, 64625 Bensheim (DE); HUELSBUSCH, Markus, 64625 Bensheim (DE); ERHARDT, Norbert, 64625 Bensheim (DE)
(74) Representative: Aldridge, Henry Alexander

(57) **Abstract**

Aspects relate to generating cephalometric x-ray images by placing a source array including a plurality of individual x-ray sources, at a first location of one side of an object to be irradiated, placing a detector including a plurality of detector areas corresponding to the plurality of individual x-ray sources at another location on another side of the object opposite the first side, and projecting through the object, during a plurality of distinct time periods, x-rays from individual x-ray sources corresponding to the distinct time periods, and combining images generated by the projections to generate a combined cephalometric x-ray image.

## Description

### BACKGROUND

### Technical Field

The present disclosure pertains to a multi beam x-ray source, and more specifically to cephalometric x-ray imaging using a source array comprising a plurality of x-ray sources and corresponding detector areas.

### Description of the Related Art

Existing methods in medical imaging have traditionally relied on projection-based techniques using one x-ray source. One of the imaging modalities include cephalometric imaging which can capture the image differently than a panoramic x-ray by using a side sweeping motion instead of the full 220 degrees non-stop motion. Further, "one shot" cephalometric systems exists, which can use a large detector and no moving parts. These systems still need a large source-to-detector distance. The cephalometric imaging may target the craniofacial region, including the teeth, jaw, skull, and soft tissues of the face, providing a comprehensive view of the relationships between these structures.

To generate cephalometric x-ray images, a large distance between the source and the detector is usually created. This may be achieved by physically disposing the source from the detector in a dental setting, requiring a predetermined minimum distance and an office setting/area with the space to allow the minimum distance to be achieved.

### BRIEF SUMMARY

Aspects relate to a method of generating cephalometric x-ray images by placing a source array including a plurality of individual x-ray sources, at a first location of one side of an object to be irradiated, placing a detector including a plurality of detector areas corresponding to the plurality of individual x-ray sources at another location on another side of the object opposite the first side, and projecting through the object, during a plurality of distinct time periods, x-rays from individual x-ray sources corresponding to the distinct time periods, and combining images generated by the projections to generate a combined cephalometric x-ray image.

In one embodiment, the images generated by the projections at different times are combined to obtain the combined x-ray image by using a weighting process wherein a portion of a first image contributes more to the combined x-ray image than a corresponding portion of a second image, the portion and the corresponding portion overlap when overlaid together. This may be performed using a plurality or all of the images taken by the individual x-ray sources and employing the weighting process to select contributions of individual images for overlapping areas such that duplication of the same information in the combined x-ray image is compensated.

Aspects further related to a cephalometric x-ray system that includes a source array includes a plurality of individual x-ray sources, the source array being disposed at a first location of one side of an object to be irradiated. The cephalometric x-ray system also includes a detector that includes a plurality of virtual or demarcated detector areas corresponding to the plurality of individual x-ray sources, the detector being disposed on another location on another side of the object opposite the first side; and a processor. The cephalometric x-ray system also includes a memory storing instructions that, when executed by the processor, configure the cephalometric x-ray system to project through the object, during a first time-period, x-rays from a first set of individual x-ray sources to a first corresponding set of detector areas, the first corresponding set of detector areas do not overlap with each other, project through the object, during a second or other time-period different from the first time period, x-rays from a second or other set of individual x-ray sources to a second or other corresponding set of detector areas, and, combine images generated by the projections from the first time period and the second and/or other time-periods to generate a combined x-ray image. The second or other corresponding set of detector areas do not overlap with each other in the set.

In one embodiment, the source array is a 1D source array comprising a (1 x N) or (N x 1) arrangement of individual x-ray sources, N being an integer greater than 1, and the source array and the detector are translated in the same direction to one or more other subsequent locations to capture one or more other parts of the object. The array may be horizontally or vertically aligned movement may be roughly perpendicular to the orientation of the source array (for a vertically aligned (1xN) source array, the array and the detector are moved horizontally and vice versa.

In an embodiment, the source array is a 2D source array comprising a (N x M) arrangement of individual x-ray sources, N and M being integer greater than 1, and a desired 2D image of the object is captured without moving the source array and/or the detector. More specifically, the 3D information is captured during the scan and projected/combined to the 2D image.

### BRIEF DESCRIPTION OF THE SEVERAL VIEWS OF THE DRAWINGS

To easily identify the discussion of any particular element or act, the most significant digit or digits in a reference number refer to the figure number in which that element is first introduced.
FIG. 1 depicts a block diagram of a network of data processing systems in accordance with an illustrative embodiment.
FIG. 2 depicts a block diagram of a data processing system in accordance with an illustrative embodiment.
FIG. 3 depicts a sketch illustrating the generation of a cephalometric x-ray image using projections from a source array of individual x-ray sources in accordance with an illustrative embodiment.
FIG. 4 depicts a sketch a 1D source array in accordance with an illustrative embodiment.
FIG. 5 depicts a sketch a 2D source array in accordance with an illustrative embodiment.
FIG. 6 depicts a sketch illustrating angles of projection in accordance with an illustrative embodiment.
FIG. 7 illustrates a routine in accordance with an illustrative embodiment.

### DETAILED DESCRIPTION

### Overview

In the following detailed description, numerous specific details are set forth by way of examples to provide a thorough understanding of the relevant teachings. However, it should be apparent that the present teachings may be practiced without such details. In other instances, well-known methods, procedures, and/or components have been described at a relatively high level, without detail, to avoid unnecessarily obscuring aspects of the present teachings.

The illustrative embodiments recognize that a cephalometric scan typically requires quasi-parallel beams that penetrate a patient's head. This may be realized by manually or automatically adjusting an x-ray system to introduce large distance between the focal spot and the patient's midsagittal plane. Typically, the distance is in the order of 1.50m. Hence, this requires much more extra space for a conventional cephalometric device in comparison to X-ray devices of other modalities.

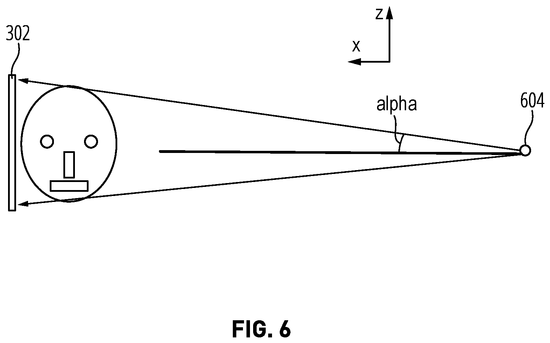

The illustrative embodiments disclose the use of a multi beam x-ray device with a multi beam x-ray source (MBXS). The multi beam x-ray device may allow the same source-to-image distance (SID) as in a PAN or CBCT device but with comparable penetration angles (alpha, See FIG. 6 which shows an x-ray source 604 from which x-rays are projected to the detector 302) as would be in a conventional cephalometric x-ray device due to methods and systems described herein. Hence, a quasi-parallel geometry as in conventional cephalometric x-ray device may be achieved, along with the same source-to-image distance (SID) as in a PAN or CBCT device, leading to the same or at least no reduction in diagnostic quality.

The illustrative embodiments are described with respect to certain types of machines. The illustrative embodiments are also described with respect to other scenes, subjects, measurements, devices, data processing systems, environments, components, and applications only as examples. Any specific manifestations of these and other similar artifacts are not intended to be limiting to the disclosure. Any suitable manifestation of these and other similar artifacts can be selected within the scope of the illustrative embodiments.

Furthermore, the illustrative embodiments may be implemented with respect to any type of data, data source, or access to a data source over a data network. Any type of data storage device may provide the data to an embodiment of the disclosure, either locally at a data processing system or over a data network, within the scope of the disclosure.

The illustrative embodiments are described using specific code, hardware, algorithms, designs, architectures, protocols, layouts, schematics, and tools only as examples and are not limiting to the illustrative embodiments. Furthermore, the illustrative embodiments are described in some instances using particular software, tools, and data processing environments only as an example for the clarity of the description. The illustrative embodiments may be used in conjunction with other comparable or similarly purposed structures, systems, applications, or architectures. For example, other comparable devices, structures, systems, applications, or architectures therefor, may be used in conjunction with such embodiment of the disclosure within the scope of the disclosure. An illustrative embodiment may be implemented in hardware, software, or a combination thereof.

The examples in this disclosure are used only for the clarity of the description and are not limiting to the illustrative embodiments. Additional data, operations, actions, tasks, activities, and manipulations will be conceivable from this disclosure and the same are contemplated within the scope of the illustrative embodiments.

Any advantages listed herein are only examples and are not intended to be limiting to the illustrative embodiments. Additional or different advantages may be realized by specific illustrative embodiments. Furthermore, a particular illustrative embodiment may have some, all, or none of the advantages listed above.

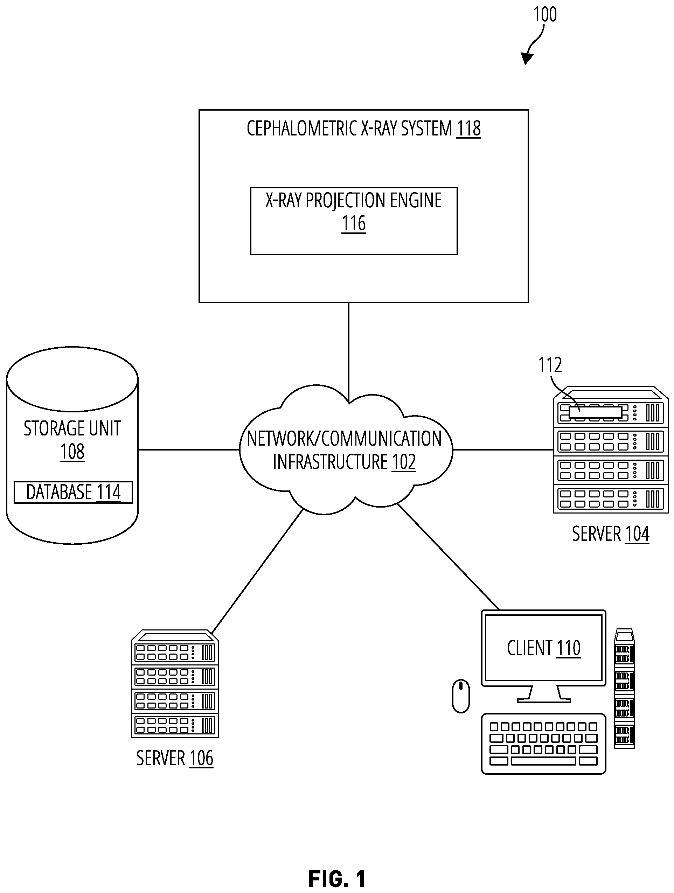

FIG. 1 depicts a block diagram of an environment of data processing systems in which illustrative embodiments may be implemented. Data processing environment 100 is a network of engines and computers in which the illustrative embodiments may be implemented. Data processing environment 100 can include network/communication infrastructure 102. Network/communication infrastructure 102 is the medium used to provide communication links between various devices, databases and computers connected together within data processing environment 100. Network/communication infrastructure 102 may include connections, such as wired connections, wireless communication protocols, or other suitable data connectivity.

The cephalometric x-ray system 118 can implement embodiments described herein. The cephalometric x-ray system 118 can include an x-ray projection engine 116 which may further include an embedded application to use data of the cephalometric x-ray system 118 for generating cephalometric x-ray radiographs. The embedded application can also execute in any of data processing systems (server 104 or server 106, client 110), such as client server application 112 in server 104.

X-ray radiography can be performed by positioning an x-ray source on one side of an object (e.g., a patient or a portion thereof) and causing the x-ray source to emit x-rays through the object and toward an x-ray detector located on the other side of the object. As the x-rays pass through the object from the x-ray source, their energies are absorbed to varying degrees depending on the composition of the object, and x-rays arriving at the x-ray detector form a two-dimensional (2D) x-ray image or projection image (also known as a radiograph) based on the cumulative absorption through the object.

Cephalometric x-ray imaging may be primarily used in dental/orthodontic/maxillofacial applications to assess the relationships between dental, skeletal, and soft tissue structures. The illustrative embodiments recognize that lateral (side view) or frontal (anterior-posterior view) images of the head may be obtained to depict the bones and facial contours in a two-dimensional plane, helping to evaluate the position of the teeth, jaw alignment, and growth patterns. In some conventional cephalometric radiographs, the X-ray source and the detector may typically be stationary with the patient's head being positioned in a fixed position using a cephalostat, and the x-ray being taken as an exposure to produce a lateral or frontal image of the head.

Turning back to FIG. 1, clients or servers are only example roles of certain data processing systems connected to network/communication infrastructure 102 and are not intended to exclude other configurations or roles for these data processing systems. Server 104 and server 106 couple to network/communication infrastructure 102 along with storage unit 108. Software applications may execute on any computer in data processing environment 100. Client 110 is also coupled to network/communication infrastructure 102. A data processing system, such as server 104 or server 106, client 110 may include data and may have software applications or software tools executing thereon.

Only as an example, and without implying any limitation to such architecture, FIG. 1 depicts certain components that are usable in an example implementation of an embodiment. As another example, an embodiment can be distributed across several data processing systems and a data network as shown, whereas another embodiment can be implemented on a single data processing system within the scope of the illustrative embodiments.

Data processing environment 100 may include additional servers, clients, and other devices that are not shown. Server 104 includes the server application 112 that may be configured to implement one or more of the functions described herein for displaying restoration proposals in accordance with one or more embodiments.

Server 106 may include a search engine configured to search stored files such as images of patient teeth for comparison in response to a request for detecting tooth defects. In the depicted example, data processing environment 100 may be the Internet. Network/communication infrastructure 102 may represent a collection of networks and gateways that use the Transmission Control Protocol/Internet Protocol (TCP/IP) and other protocols to communicate with one another. FIG. 1 is intended as an example, and not as an architectural limitation for the different illustrative embodiments.

Among other uses, data processing environment 100 may be used for implementing a client-server environment in which the illustrative embodiments may be implemented. A client-server environment enables software applications and data to be distributed across a network such that an application functions by using the interactivity between a client data processing system and a server data processing system. Data processing environment 100 may also employ a service-oriented architecture where interoperable software components distributed across a network may be packaged together as coherent business applications. Data processing environment 100 may also take the form of a cloud, and employ a cloud computing model of service delivery for enabling convenient, on-demand network access to a shared pool of configurable computing resources (e.g. networks, network bandwidth, servers, processing, memory, storage, applications, virtual machines, and services) that can be rapidly provisioned and released with minimal management effort or interaction with a provider of the service.

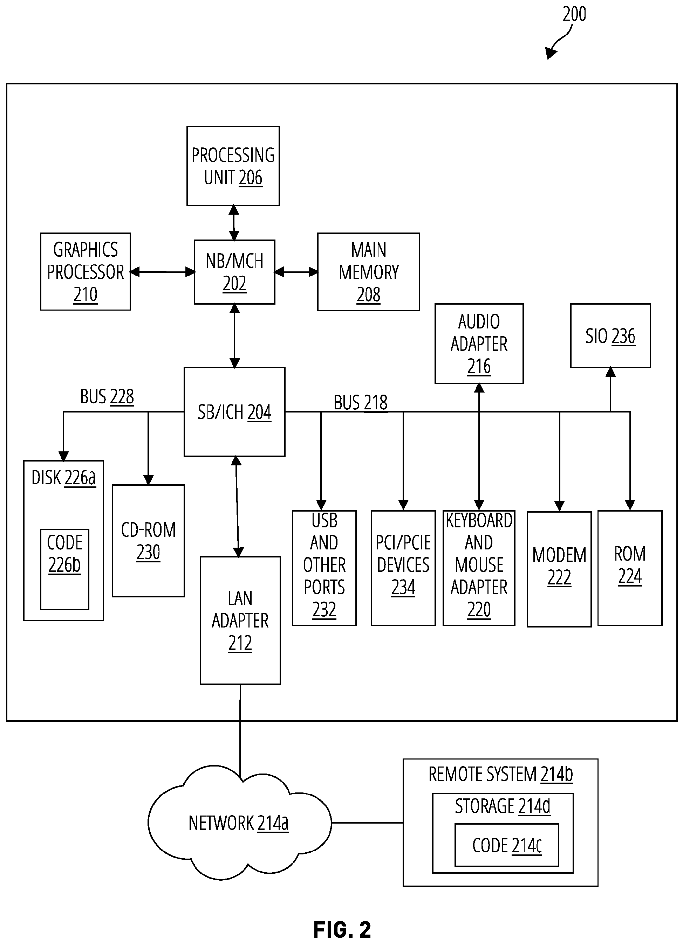

With reference to FIG. 2, this figure depicts a block diagram of a data processing system in which illustrative embodiments may be implemented. Data processing system 200 is an example of a computer, such cephalometric x-ray system 118, client 110 or server 104 in FIG. 1, or another type of device in which computer usable program code or instructions implementing the processes may be located for the illustrative embodiments.

Data processing system 200 is described as a computer only as an example, without being limited thereto. Implementations in the form of other devices, may modify data processing system 200, such as by adding a touch interface, and even eliminate certain depicted components from data processing system 200 without departing from the general description of the operations and functions of data processing system 200 described herein.

In the depicted example, data processing system 200 employs a hub architecture including North Bridge and memory controller hub (NB/MCH) 202 and South Bridge and input/output (I/O) controller hub (SB/ICH) 204. Processing unit 206, main memory 208, and graphics processor 210 are coupled to North Bridge and memory controller hub (NB/MCH) 202. Processing unit 206 may include one or more processors and may be implemented using one or more heterogeneous processor systems. Processing unit 206 may be a multicore processor. Graphics processor 210 may be coupled to North Bridge and memory controller hub (NB/MCH) 202 through an accelerated graphics port (AGP) in certain implementations.

In the depicted example, local area network (LAN) adapter 212 is coupled to South Bridge and input/output (I/O) controller hub (SB/ICH) 204. Audio adapter 216, keyboard and mouse adapter 220, modem 222, read only memory (ROM) 224, universal serial bus (USB) and other ports 232, and PCI/PCIe devices 234 are coupled to South Bridge and input/output (I/O) controller hub (SB/ICH) 204 through bus 218. Hard disk drive (HDD) or solid-state drive (SSD) 226a and CD-ROM 230 are coupled to South Bridge and input/output (I/O) controller hub (SB/ICH) 204 through bus 228. PCI/PCIe devices 234 may include, for example, Ethernet adapters, add-in cards, and PC cards for notebook computers. PCI uses a card bus controller, while PCIe does not. Read only memory (ROM) 224 may be, for example, a flash binary input/output system (BIOS). Hard disk drive (HDD) or solid-state drive (SSD) 226a and CD-ROM 230 may use, for example, an integrated drive electronics (IDE), serial advanced technology attachment (SATA) interface, or variants such as external-SATA (eSATA) and micro-SATA (mSATA). A super I/O (SIO) device 236 may be coupled to South Bridge and input/output (I/O) controller hub (SB/ICH) 204 through bus 218.

Memories, such as main memory 208, read only memory (ROM) 224, or flash memory (not shown), are some examples of computer usable storage devices. Hard disk drive (HDD) or solid-state drive (SSD) 226a, CD-ROM 230, and other similarly usable devices are some examples of computer usable storage devices including a computer usable storage medium.

An operating system runs on processing unit 206. The operating system coordinates and provides control of various components within data processing system 200 in FIG. 2. The operating system may be a commercially available operating system for any type of computing platform, including but not limited to server systems, personal computers, and mobile devices. An object oriented or other type of programming system may operate in conjunction with the operating system and provide calls to the operating system from programs or applications executing on data processing system 200.

Instructions for the operating system, the object-oriented programming system, and applications or programs, such as server application 112 in FIG. 1, are located on storage devices, such as in the form of codes 226b on Hard disk drive (HDD) or solid-state drive (SSD) 226a, and may be loaded into at least one of one or more memories, such as main memory 208, for execution by processing unit 206. The processes of the illustrative embodiments may be performed by processing unit 206 using computer implemented instructions, which may be located in a memory, such as, for example, main memory 208, read only memory (ROM) 224, or in one or more peripheral devices.

Furthermore, in one case, code 226b may be downloaded over network 214a (such as network/communication infrastructure 102) from remote system 214b, where similar code 214c is stored on a storage device 214d in another case, code 226b may be downloaded over network 214a to remote system 214b, where downloaded code 214c is stored on a storage device 214d.

A communications unit may include one or more devices used to transmit and receive data, such as a modem or a network adapter. A memory may be, for example, main memory 208 or a cache, such as the cache found in North Bridge and memory controller hub (NB/MCH) 202. A processing unit may include one or more processors or CPUs.

Where a computer or data processing system is described as a virtual machine, a virtual device, or a virtual component, the virtual machine, virtual device, or the virtual component operates in the manner of data processing system 200 using virtualized manifestation of some or all components depicted in data processing system 200. For example, in a virtual machine, virtual device, or virtual component, processing unit 206 is manifested as a virtualized instance of all or some number of hardware processing units 206 available in a host data processing system, main memory 208 is manifested as a virtualized instance of all or some portion of main memory 208 that may be available in the host data processing system, and Hard disk drive (HDD) or solid-state drive (SSD) 226a is manifested as a virtualized instance of all or some portion of Hard disk drive (HDD) or solid-state drive (SSD) 226a that may be available in the host data processing system. The host data processing system in such cases is represented by data processing system 200.

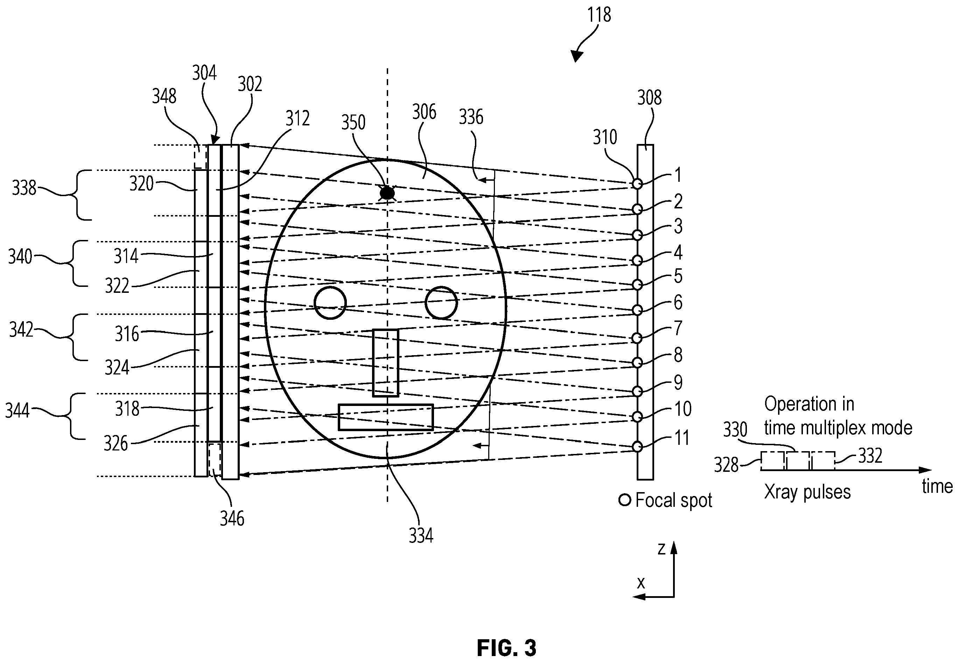

FIG. 3 shows a sketch of an operation of a cephalometric x-ray system 118 in accordance with an illustrative embodiment. The cephalometric x-ray system 118 is a multi-beam x-ray device that comprises a source array 308 including a plurality of individual x-ray sources 310. The source array 308 is disposed at a first location of one side of an object 306 to be irradiated.

The cephalometric x-ray system 118 also comprises a detector 302 which is physically or virtually demarcated to have a plurality of detector areas 304 each corresponding to an individual x-ray source 310 of the plurality of individual x-ray sources 310. The individual x-ray sources may be nanotubes, for example carbon nanotubes. Other examples of the x-ray sources may be cold cathode emitters which are not electrically heated by a filament and instead may extract electrons from metal by an external electric field. The sources may also be coupled with collimators configured to limit x-ray beams. Though the detector areas 304 are a part of the detector 302, the detector areas 304 have been shown outside and adjacent to the detector 302 to illustrate the parts of the detector 302 corresponding to the detector areas 304. The detector 302 is disposed at another location on another side of the object 306, the another side being opposite the first side. In an aspect, the distance between the source array 308 and detector can be about 0.5m (e.g., +/- 1%, 5%, or 10%, or 20%), or a distance from 0.3m to 1.2m. The midsagittal plane 334 of the object may be as close as possible to the detector. In other aspects, the distance is smaller than 1.2m. Alternatively, the same distance as for PAN or CBCT can be used. More specifically, if the device can perform CEPH, PAN and/or CBCT, the same detector and source array with the same setup for all modalities can be used. Even further, the source-to-detector distance may be a factor of a dimension of to the demarcated detector areas 304. e.g. the SID is less than 8 * largest length (height) of demarcated detector area 304.

In an aspect, the cephalometric x-ray system 118 is configured to project through the object 306, during a first time period 328, x-rays from a first set ( e.g. 1, 4, 7, and 10 as shown in FIG. 3) of individual x-ray sources 310 to a first corresponding set of detector areas 304 (detector area A 312, detector area B 314, detector area C 316, detector area D 318), the first corresponding set of detector areas do not overlap with each other in the set. More specifically, the detector areas are not meant to overlap with each other though in theory irrelevant portions of the x-rays of one x-ray source such as scatter radiation can hit the detector area corresponding to another x-ray source.

The cephalometric x-ray system 118 is also configured to project through the object 306, during a second time period 330 different from the first time period 328, x-rays from a second set (2, 5, 8, 11 as shown in FIG. 3) of individual x-ray sources 310 to a second corresponding set of detector areas (detector area E 320, detector area F 322, detector area G 324, detector area H 326), the second corresponding set of detector areas do not overlap with each other in the set;

The cephalometric x-ray system 118 may further be configured to project through the object 306, during an other time period 332 (such as a third time period) different from the first time period 328 or the second time period 330, x-rays from an other set (e.g., 3, 6, 9 ... as shown in FIG. 3) of individual x-ray sources 310 to another corresponding set of detector areas (not shown), the other corresponding set of detector areas do not overlap with each other in the set. Of course, these are not meant to be limiting as similar implementations may be obtained in view of the descriptions herein. For example, the first set of individual x-ray sources and the first corresponding set of detector areas can include at least one individual x-ray source and at least one corresponding detector area, respectively. Likewise, the second or other set of first set of individual x-ray sources x-ray sources and the second or other corresponding set of detector areas can include at least one x-ray source and at least one corresponding detector area, respectively.

The cephalometric x-ray system 118 may further be configured to combine images generated by the projections from the first time period and the second and/or other time-periods to generate a combined x-ray image.

Advantageously, due to the use of multiple individual x-ray sources 310 which are relatively small in comparison to conventional sources, relatively parallel (quasi-parallel) projected rays can be obtained without introducing conventional distances between the source and the midsagittal plane 334. More specifically, the illustrative embodiments recognize that to cover the full head, a large cone/fan beam angle may be used or this can be compensated by using a smaller cone/fan beam angle with a larger distance between x-ray source and midsagittal plane. However, by using multiple x-ray sources, each x-ray source may simply have to cover a small part of the head. The more x-ray sources there are in one direction, the smaller is the part of the head to cover, and therefore the smaller the cone/fan angle or the smaller the distance between x-ray sources and midsagittal plane. The closer the object is to the detector, the more likely information about the object can be obtained. Thus, in some embodiments, an information area 336 may be defined that depicts where complete information about the object can be obtained when the object is placed inside that area.

Further, at least some of the detector areas 304 used during the first time period 328 overlap with at least some of the detector areas 304 used during the second time period 330 or other time period 332. The overlapping helps prevent or alleviate the issue of missing information about the object 306 that can be caused by missed spots on the detector. Further, the extent of the desired information area 336 and the geometrical setup of the x-ray sources and the distance between x-ray sources and the detector give a limit to the use or size of the overlapping detector areas. To get information of the distance of the information area from the detector, overlapping of the areas on the detector may be helpful. In an aspect, to facilitate the overlapping, each detector area of the plurality of detector areas used in a time period may receive x-ray projections at some portion thereof during other time periods. For example, some portions of each detector area 312, 314, 316, and 318 used in the first time period 328 also receive projections in the second time period 330 through 320, 322, 324, and 326. By so doing, overlapping areas such as, but not limited to, overlapping areas 338, 340, 342, and 344 may be generated.

However, by overlapping the areas, duplicated information about the object may be obtained, though a selection or weighting algorithm may be used to fully or partially select contributions from individual duplicates for the combined x-ray image (for example at a pixel level). In the design where there are overlapping areas, since the overlapping areas allow for depth-estimation of structures, a limited angle reconstruction can be performed. A limited angle reconstruction does not require full information from all angles and may thus be possible with sparse angular information from limited angle range. By imaging an object from more than one perspective (x-ray source position) with known geometry (x-ray source positions and orientation, and detector positions and orientation are known), the 3d position of an imaged structure can be estimated. This reconstruction can be projected on the midsagittal plane 334 to reduce artifacts.

An area (in the device) in which the structures are completely imaged can be defined. This area takes into account the common patient positioning and defines the position of the individual x-ray sources to minimize the overlapping areas. Areas of the detector that are not irradiated in a time period, for example unused areas 346, 348, can be used for scatter estimation. More specifically, when an x ray penetrates a human's head, three effects may be possible. A first effect includes no interaction wherein the x-ray travels through parts of head and hits the detector 302. A second effect, the photo effect, includes absorption of the x-ray, and the third effect comprises "Compton scattering", where the x-ray is scattered. Thus, unused areas 346, 348, can be used to detect scattered x-rays.

In a further aspect, the individual x-ray source 310 may be arranged such that the projected images do not overlap with each other. Therefore, the projected images can be added together to generate the combined x-ray image.

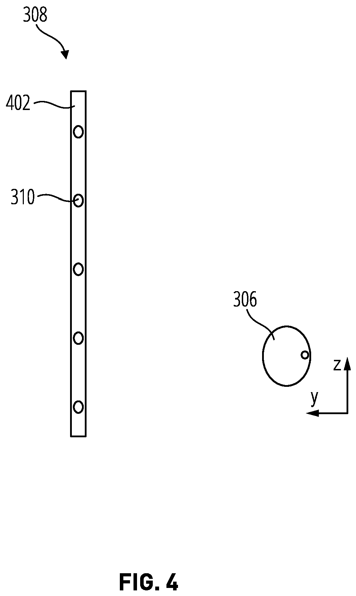

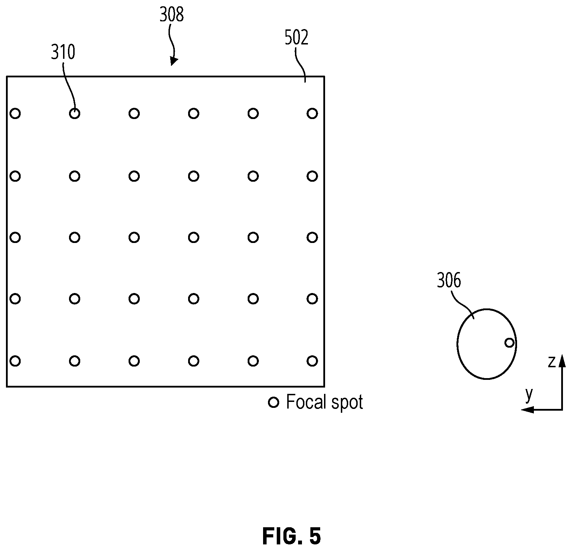

Turning now to FIG. 4 and FIG. 5, different implementations of the source array 308 are illustrated. FIG. 4 shows a 1D source array 402 comprising a (1 x N) or (N x 1) arrangement of individual x-ray sources 310, N being an integer greater than 1. The corresponding detector 302 for the 1D source array 402 may be dimensioned to have just enough area to capture the projected x-rays of the 1D source array 402. When the 1D source array 402 is used with the detector 302 for a first projection shot, the 1D source array 402 and the detector 302 are translated together in the same direction to one or more other subsequent locations to capture one or more other projection shots. The translation is performed such that all relevant parts of the object 306 that have to be captured to cover a total desired information area 336 of the object are captured. Further, additional rotational motion may be added in embodiments if needed.

FIG. 5 illustrates another implementation comprising a 2D source array 502 comprising an (N x M) arrangement of individual x-ray sources 310, N and M being integer greater than 1. The corresponding detector 302 for the 2D source array 502 may be dimensioned to have just enough area to capture the projected x-rays of the 2D source array 502. Due to the comparatively larger area of the 2D source array 502 relative to the 1D source array 402, translating the 2D source array 502 and its corresponding detector may be obviated because the 2D source array 502 may be large enough to cover the total desired volume or information area 336 of the object that is being studied. Thus, a single projection shot may be enough. Overlapping detector areas / x-ray sources would be operated in time multiplex. Of course, this is not meant to be limiting as a 2D source array 502 that is comparatively smaller than the desired volume being studied may be moved a plurality of times along with the corresponding detector to capture the entire desired volume. Even further, 3D arrays may be realized wherein there the sources of the 3D array are arranged at different depths/distances from the detector.

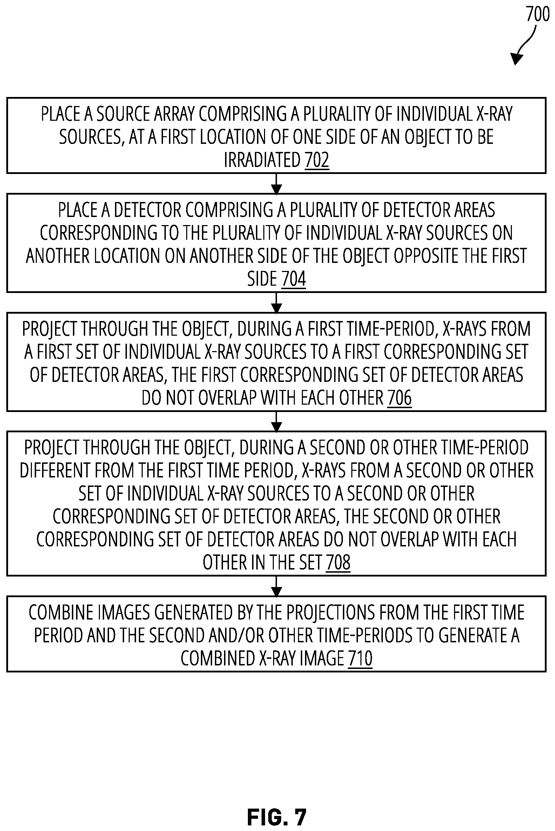

Turning now to FIG. 7, a routine 700 for multi-beam projection is disclosed. At least a portion of the routine 700 may be performed with a cephalometric x-ray system 118, x-ray projection engine 116 or an application thereof.

In block 702, a source array 308 is provided comprising a plurality of individual x-ray sources 310, at a first location of one side of an object 306 to be irradiated. In block 704, a detector 302 comprising a plurality of detector areas 304 corresponding to the plurality of individual x-ray sources 310 is provided at another location on another side of the object 306 the another side being opposite to the first side. In block 706, the x-ray projection engine 116 projects through the object 306, during a first time period 328, x-rays from a first set of individual x-ray sources to a first corresponding set of detector areas, the first corresponding set of detector areas do not overlap with each other. In block 708, the x-ray projection engine 116 projects through the object 306, during a second or other time-period different from the first time period, x-rays from a second or other set of individual x-ray sources to a second or other corresponding set of detector areas. In block 710, routine 700 the projection images or radiographs generated by the projections from the first time period and the second and/or other time-periods are combined to generate a combined x-ray image.

In an aspect, the combination is performed by using a weighting process wherein a portion of one image contributes more to the combined x-ray image than a corresponding portion of one or more other images. The portions are portions that overlap and may therefore be duplicates or multiple duplicates each having slightly different information. In another aspect, a neural network can be trained to generate the combination by using input training individual images and an output combined image that have corresponding portions of the input automatically selected for representation in the combined image based on a predetermined criteria. By using a large number of these training inputs and corresponding training output for training and a subset for testing, the neural network can be trained to perform the combination automatically using a new set of unseen input images/radiographs.

In an even further aspect, combined images may be generated using virtual planes in the object or outside the object such as between the object and the detector wherein virtual radiographs are simulated for certain predetermined virtual planes and a combined x-ray image is created by combining the virtual radiographs. This may have the effect of better representing particular anatomical structures that coincide with the virtual planes better in the combined x-ray image or to reduce artifacts. For example, consider point X 350 in the midsagittal plane 334 of the object 306 of FIG. 3. In the illustration, point X 350 may be imaged two times (for example) by individual x-ray sources 1 and 2 and thus may appear two times on the detector 302, with the relative distances between them depending on the geometric arrangement of individual x-ray sources 1 and 2. If the plane inside the object 306 and point X 350 are shifted more towards the source (*i.e.*, no longer the midsagittal plane 334), then said distance between the representation of point X 350 on the detector 302 will become larger. Likewise, if the plane and point X 350 are moved towards the detector, the distance will be smaller. Using the plurality of images generated from the plurality of individual x-ray sources, a reconstruction taking into account the relative distances can be performed to generate virtual radiographs in any virtual plane of the object.

More generally, alternative combined x-ray images (CEPH-like images) for virtual planes can be generated. Generating the combined images is based on the depth information of the virtual plane (the distance from the x-ray sources, i.e., the virtual planes lie between the sources and the detector) and the projections, wherein the detector position can be considered as a default virtual plane. Additionally, the projection geometry may be used, which includes the geometrical setup of the source array (x-ray source positions and orientation) and the position and orientation of the detector. The projection geometry is known from hardware design or device calibration. For the virtual plane the position and orientation can be defined. When the virtual plane is varied, it may vary the depth (depth information). Further, a virtual volume may be generated, and one or more corresponding combined x-ray images are generated based at least on information of about the virtual volume and a projection geometry. More specifically, a virtual volume may be generated from the projections (and the projection geometry). From this virtual volume one or more combined x-ray images are generated, e.g. by projecting the volume information to corresponding virtual planes or by slicing the volume by virtual planes.

In one aspect, further overlapping areas may be generated in the same time period and thus the image generated on the detector can include mixed information corresponding to different individual x-ray sources. Thus, a decomposition process can be used to decompose the mixed information using a trained neural network. The trained neural network can be trained using images that contain mixed information from different sources and corresponding individual images from the different sources that would have been produced if there were no mixing. By using a large number of these as training inputs and training outputs, the neural network can be trained to decompose input mixed images into their individual non-mixed images and tested for deployment.

By methods and systems described herein, cephalometric x-ray images/radiographs can be obtained without the use of a conventional "CEPH" arm or a need to change the SID since the SID of PAN / CBCT device can be used.

Thus, a computer implemented method, system or apparatus, and computer program product are provided in the illustrative embodiments for cephalometric x-ray imaging and other related features, functions, or operations. Where an embodiment or a portion thereof is described with respect to a type of device, the computer implemented method, system or apparatus, the computer program product, or a portion thereof, are adapted or configured for use with a suitable and comparable manifestation of that type of device.

The present invention may be a system, a method, and/or a computer program product at any possible technical detail level of integration. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, including but not limited to computer-readable storage devices as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

These computer readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method of generating cephalometric x-ray images comprising:
placing a source array comprising a plurality of individual x-ray sources, at a first location of one side of an object to be irradiated;
placing a detector comprising a plurality of detector areas corresponding to the plurality of individual x-ray sources at another location on another side of the object opposite the first side;
projecting through the object, during a first time period, x-rays from a first set of individual x-ray sources to a first corresponding set of detector areas, the first corresponding set of detector areas do not overlap with each other;
projecting through the object, during a second or other time-period different from the first time period, x-rays from a second or other set of individual x-ray sources to a second or other corresponding set of detector areas, the second or other corresponding set of detector areas do not overlap with each other in the set;
combining images generated by the projections from the first time period and the second and/or other time-periods to generate a combined x-ray image.

2. The method of claim 1, further comprising, providing a distance between the source array and the detector from 0.3m to 1.2m.

3. The method of claim 1 or claim 2, wherein the combining further includes:
using a weighting process wherein a portion of a first image and a corresponding portion of a second image both contribute equally or unequally to the combined x-ray image,
wherein the portion overlaps with the corresponding portion when the first image and second image are overlaid together.

4. The method of any of claims 1-3, further comprising:
generating at one or more virtual planes, one or more corresponding combined x-ray images, based at least on depth information of the one or more virtual planes and a projection geometry; or
generating a virtual volume, and one or more corresponding combined x-ray images, based at least on volume information about the virtual volume and a projection geometry.

5. The method of any of claims 1-4, wherein:
the first set of individual x-ray sources and the first corresponding set of detector areas are set up to include at least one individual x-ray source and at least one corresponding detector area, respectively; and/or
the second or other set of individual x-ray sources and the second or other corresponding set of detector areas are set up to include at least one individual x-ray source and at least one corresponding detector area, respectively.

6. The method of any of claims 1-5, further comprising:
translating the source array and the detector in the same direction to one or more other subsequent locations to capture one or more other parts of the object, wherein the source array is a 1D source array comprising a (1 x N) or (N x 1) arrangement of individual x-ray sources, N being an integer greater than 1; or
capturing a desired volume of the object without moving the source array and/or detector, wherein the source array is a 2D source array comprising a (N x M) arrangement of individual x-ray sources, N and M being integer greater than 1.

7. The method of any of claims 1-6, further comprising:
using an area of the detector that is not directly irradiated to compensate for scatter radiation in the combined x-ray image.

8. A cephalometric x-ray system comprising:
a source array comprising a plurality of individual x-ray sources, the source array being disposed at a first location of one side of an object to be irradiated;
a detector comprising a plurality of detector areas corresponding to the plurality of individual x-ray sources, the detector being disposed on another location on another side of the object opposite the first side;
a processor; and
a memory storing instructions that, when executed by the processor, configure the cephalometric x-ray system to:
project through the object, during a first time-period, x-rays from a first set of individual x-ray sources to a first corresponding set of detector areas, the first corresponding set of detector areas do not overlap with each other;
project through the object, during a second or other time-period different from the first time period, x-rays from a second or other set of individual x-ray sources to a second or other corresponding set of detector areas, the second or other corresponding set of detector areas do not overlap with each other in the set;
combine images generated by the projections from the first time period and the second and/or other time-periods to generate a combined x-ray image.

9. The cephalometric x-ray system of claim 8, wherein a distance between the source array and the detector is from 0.3m to 1.2m.

10. The cephalometric x-ray system of claim 8 or 9, wherein the plurality of individual x-ray sources are nanotubes or cold cathode emitters.

11. The cephalometric x-ray system of any of claims 8-10, wherein at least some of the detector areas used during the first time-period overlap with at least some of the detector areas used during the second or other time period.

12. The cephalometric x-ray system of any of claim 8-11, wherein:
the first set of individual x-ray sources and the first corresponding set of detector areas include at least one individual x-ray source and at least one corresponding detector area, respectively; and/or
the second or other set of x-ray sources and the second or other corresponding set of detector areas include at least one individual x-ray source and at least one corresponding detector area, respectively.

13. The cephalometric x-ray system of any of claims 8-12, wherein:
the source array is a 1D source array comprising a (1 x N) or (N x 1) arrangement of x-ray sources, N being an integer greater than 1, and the processor is further configured to translate the source array and the detector in the same direction to one or more other subsequent locations to capture one or more other parts of the object; or
the source array is a 2D source array comprising a (N x M) arrangement of x-ray sources, N and M being integer greater than 1, and the processor is further configured to capture a desired volume of the object without moving the source array and/or detector during capturing.

14. A non-transitory computer-readable storage medium including instructions that when executed by a computer, cause the computer to:
project through an object, during a first time-period, x-rays from a first set of individual x-ray sources to a first corresponding set of detector areas, the first corresponding set of detector areas do not overlap with each other;
project through the object, during a second or other time-period different from the first time period, x-rays from a second or other set of individual x-ray sources to a second or other corresponding set of detector areas, the second or other corresponding set of detector areas do not overlap with each other in the set;
combine images generated by the projections from the first time period and the second and/or other time-periods to generate a combined x-ray image.

15. The non-transitory computer-readable storage medium of claim 14, wherein at least some of the detector areas used during the first time-period are configured to overlap with at least some of the detector areas used during the second or other time period.
